# FASCICULE DE BREVET EUROPEEN

(11) **EP 2 343 971 B1**
(45) Date de publication et mention de la délivrance du brevet: **13.05.2015**
(21) Numéro de dépôt: 09740447.9
(22) Date de dépôt: 07.08.2009
(51) Int. Cl.: A01N 25/00, A61K 9/08, A61K 47/02, A61K 47/10, A61K 9/00

(54) **SOLUTION ANTISEPTIQUE INCOLORE A USAGE CUTANE**
FARBLOSE ANTISEPTISCHE LÖSUNG ZUR VERWENDUNG AUF DER HAUT
COLORLESS ANTISEPTIC SOLUTION FOR SKIN USE

(30) Priorité: 11.08.2008 FR 0855516
(43) Date de publication de la demande: 20.07.2011
(73) Titulaire: Unither Developpement, 80080 Amiens (FR)
(72) Inventeur: MAURY, Marc, F-33160 Saint Medard en Jalles (FR)
(74) Mandataire: Cenatiempo, Julie Adeline Anne
(86) Numéro de dépôt international: PCT/FR2009/051571
(87) Numéro de publication internationale: WO 2010/018339

(56) Documents cités:
- EP-A- 0 934 742
- EP-A- 1 044 686
- WO-A-97/02038
- WO-A-2004/083905
- WO-A-2007/130982
- WO-A-2008/060355
- FR-A- 2 831 434
- US-A- 5 837 645
- STOILOVIC ET AL: "Improved method for DFO development of latent fingerprints" FORENSIC SCIENCE INTERNATIONAL, ELSEVIER SCIENTIFIC PUBLISHERS IRELAND LTD, IE, vol. 60, no. 3, 1 août 1993 (1993-08-01), pages 141-153, XP023110761 ISSN: 0379-0738

## Description

La présente invention concerne une solution antiseptique à usage cutané, originellement incolore par sa composition et contenant une substance colorante permettant de visualiser son application effective sur la peau. La coloration ne survient qu'au contact de la solution avec la peau.

L'invention se rapporte également à un procédé de préparation d'une telle solution ainsi qu'à son utilisation.

Les antiseptiques sont des produits qui tuent ou préviennent la croissance de microorganismes sur les surfaces externes du corps.

Lorsque de tels produits sont appliqués sur la peau, notamment en milieu hospitalier, par exemple avant la pose de matériel de perfusion, de cathéter ou plus particulièrement lors de la préparation des champs opératoires, il est nécessaire de pouvoir vérifier aisément que la zone concernée a bien été aseptisée.

Les recommandations de bonnes pratiques d'utilisation des antiseptiques en milieu hospitalier, préconisent notamment, lors d'une intervention chirurgicale, d'appliquer préalablement à toute incision une solution antiseptique qui colore le champ opératoire. Le chirurgien peut alors visualiser et vérifier la zone d'application du produit sur la peau, limitant ainsi les risques d'apparition d'infections. Le marquage de la peau est également un traceur qui permet de justifier du respect des procédures de désinfection après l'opération. En effet dans le cas ou une surinfection intervient après l'opération, il est important pour l'équipe chirurgicale de pouvoir prouver que les opérations de désinfection ont bien été réalisées dans les règles. Dans ce cas le marquage de la peau doit pouvoir être rémanent sur plusieurs jours.

Actuellement, pour répondre aux préconisations de bonnes pratiques, on peut utiliser des solutions antiseptiques à base d'iode qui sont naturellement colorées, telles que le produit connu sous la dénomination Bétadine® champ.

Toutefois, si les dérivés iodés permettent bien de visualiser la zone aseptisée, ils présentent malheureusement des risques d'allergie à l'iode chez certains sujets et des risques d'intoxication à l'iode lors d'usages répétés. Ces inconvénients rendent nécessaire la mise à disposition des praticiens d'autres solutions antiseptiques basées sur des molécules actives différentes.

On connaît par ailleurs des produits antiseptiques à l'efficacité démontrée, tels que la Chlorhexidine ou le Benzalkonium. Ce sont généralement des molécules à caractère anionique ou cationique qui présentent des interactions fréquentes avec les molécules colorantes, rendant difficile la formulation de solutions antiseptiques stables. Soit les interactions provoquent l'apparition de précipités, soit elles induisent une perte d'activité antiseptique rendant ces formulations incertaines en terme d'efficacité antiseptique.

C'est pourquoi, en pratique, la quasi-totalité des formulations antiseptiques colorées à application cutanée, autres que celles à base d'iode, sont présentes sous forme de plusieurs solutions avec préparation extemporanée de la coloration.

A titre d'exemple on peut citer la formulation commercialisée sous la dénomination Hibitane^{®} champ, composée de deux solutions : une première solution contenant de la chlorhexidine digluconate et une seconde solution contenant de l'azorubine, un colorant de couleur rouge.

Les solutions doivent être mélangées extemporanément avant usage, ce qui complique considérablement le travail du médecin et de ses assistants.

En outre, même avec ces préparations, l'interaction entre les composants antiseptiques et les composés colorés induit une réduction de la capacité antiseptique de ces mêmes préparations obligeant à accroître la quantité de préparation utilisée pour compenser la fraction susceptible de réagir avec les composants colorés.

Pour éviter ces problématiques, une solution a été proposée, notamment dans la demande de brevet FR-2791890 qui décrit une méthode de préparation de solutions antiseptiques colorées prêtes à l'emploi, ne nécessitant pas de mélange extemporané avant utilisation.

Toutefois, les produits décrits dans cette demande ne sont pas satisfaisants car, malgré les précautions prises, une perte en substance colorée peut apparaître dans la formulation lors de la fabrication, rendant de fait complètement aléatoire toute application industrielle.

Aussi, la présente invention vise à pallier les inconvénients de l'art antérieur en proposant une solution antiseptique incolore prête à l'emploi, stable, permettant de stigmatiser de manière fiable l'application antiseptique sur la peau.

A cet effet, l'invention a pour objet une solution antiseptique à usage cutané comprenant au moins un principe actif antiseptique, de l'alcool et au moins une substance capable d'induire une coloration immédiate et/ou retardée de la peau. L'invention concerne également un procédé de préparation de cette solution antiseptique et son utilisation.

Avantageusement, lorsque la solution antiseptique selon l'invention est appliquée sur la peau, cela engendre une coloration cutanée qui permet de témoigner de l'application du produit et de vérifier que la zone concernée a bien été aseptisée. Selon un autre aspect, la présente invention couvre également un procédé de préparation d'une solution antiseptique à usage cutané.

Par usage cutané on entend le dépôt de la solution par un moyen approprié tel que des compresses ou par pulvérisation sur la peau.

Par coloration de la peau ou coloration cutanée on entend un contraste de coloration de la peau évident à l'oeil de l'observateur par rapport aux zones cutanées non traitées. Cette manifestation peut prendre une coloration marron ou brunâtre, plus ou moins intense.

Un autre avantage lié au caractère incolore de la solution est l'absence de colorations accidentelles dommageables et coûteuses pour les installations, pour les matériels médicaux et chirurgicaux.

L'invention est maintenant décrite en détail.

### CARACTERISATION DE LA SOLUTION ANTISEPTIQUE A USAGE CUTANEE

La solution antiseptique incolore à usage cutané selon l'invention comprend au moins un principe actif antiseptique cutané, de l'alcool et au moins une substance incolore capable d'induire une coloration de la peau, de façon immédiate et/ou retardée.

Aussi, c'est l'association particulière agent antiseptique, alcool, substance incolore colorant la peau de façon immédiate et/ou retardée qui confère ses caractéristiques particulièrement avantageuses à la solution antiseptique selon l'invention.

On entend pour la suite de la description par coloration de la peau, une coloration particulière différente de la couleur naturelle de la peau mais également tout contraste instantanément différenciable de l'état initial.

La substance capable d'induire une coloration de la peau est choisie parmi toutes les substances incolores capables de colorer la peau et ne présentant pas de fonction chimique susceptible d'incompatibilité avec les actifs antiseptiques. De manière préférée la substance incolore capable d'induire une coloration de la peau est une substance incolore telle que, par exemple, la Dihydroxyacétone, la Ninhydrine ou un de ses sels, un sel d'argent (nitrate) ou de la D.F.O (1,8-diaza-9-fluorénone). Il peut s'agir également d'une encre fluorescente.

Selon un mode de réalisation particulièrement adapté, la substance capable d'induire une coloration de la peau est la Dihydroxyacétone, de formule :

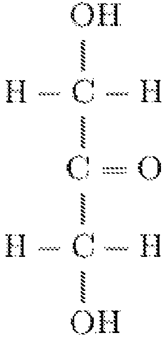

La Dihydroxyacétone est une substance capable de réagir sélectivement avec la couche superficielle des cellules mortes de la peau par une réaction de Maillard avec ses composants kératineux. Elle donne à la peau une coloration temporaire brune.

Préférentiellement, la Dihydroxyacétone est présente dans la solution à une concentration comprise entre 0,1% et 10% en poids de la solution selon l'invention, encore plus préférentiellement entre 1 et 5%.

La coloration peut être obtenue avec d'autres molécules réagissant avec la peau. Par exemple la Ninhydrine réagit avec les acides aminés des protéines de la peau en donnant une coloration brun à brun pourpre. Elle peut être présente dans la solution selon l'invention à une concentration comprise entre 0,05 et 2%.

Il peut s'agir également de la D.F.O (1,8-diaza-9-fluorénone), préférentiellement présente dans la solution selon l'invention à une concentration comprise entre 0,1 et 5%.

De la même façon, il est également possible d'obtenir le même résultat en utilisant un sel d'argent comme du nitrate d'argent qui, au contact de la peau, forme un chlorure d'argent, qui lui-même précipite sous l'effet de la lumière sous forme d'argent métallique, donnant à la peau une coloration marron à gris marron. Le sel d'argent peut être présent dans la solution selon l'invention à une concentration comprise entre 0,1 et 2%.

Selon une autre variante, la substance incolore capable d'induire une coloration de la peau utilisée dans la solution selon l'invention peut être une encre fluorescente, préférentiellement présente dans la solution selon l'invention à une concentration comprise entre 0,1 et 5%. Cette coloration permet de faire ressortir immédiatement sous l'effet d'une lampe UV un marquage de la zone désinfectée, puis ultérieurement un marquage retardé afin de vérifier que la zone a bien été aseptisée.

De façon préférée, le principe actif antiseptique est présent dans la solution à une concentration comprise entre 0,05 et 5%, en particulier entre 0,1 et 1%. Ce principe actif antiseptique peut être choisi parmi la Chlorhexidine base, l'Hexamidine et/ou le Benzalkonium ou un de leurs sels.

La présence d'un alcool dans la solution antiseptique selon la présente invention joue un rôle essentiel :
- qualité antimicrobienne propre,
- l'alcool assure la dissolution du film lipidique cutané permettant ainsi à cet alcool et aux autres principes actifs d'exercer leur action bactéricide en profondeur, en désorganisant le substrat sur lequel adhèrent les agents infectieux,
- l'alcool permet simultanément le passage de l'agent colorant indicateur d'asepsie à travers ce même film lipidique et donc le marquage rapide de la couche dermique.

Préférentiellement la concentration en alcool est comprise entre 30 et 85%.

L'alcool peut être choisi par exemple parmi les alcools éthyliques ou isopropyl iques.

### PROCEDE DE PREPARATION

La solution antiseptique à usage cutané selon l'invention peut être obtenue par un procédé de préparation comprenant au moins les étapes suivantes :
- mélanger de l'eau purifiée et de l'alcool,
- ajouter une substance incolore capable d'induire une coloration de la peau et agiter jusqu'à dissolution complète, et
- ajouter au moins un principe actif antiseptique et agiter jusqu'à dissolution complète.

Préférentiellement le procédé comprend une étape supplémentaire de filtration.

### UTILISATIONS

La solution selon l'invention peut être utilisée pour aseptiser les surfaces externes du corps.

Elle est particulièrement utile en milieu hospitalier pour tuer et/ou prévenir la croissance de microorganismes sur les surfaces externes du corps avant toute intervention avec effraction cutanée, notamment une intervention chirurgicale. Lorsque la solution selon l'invention est appliquée sur la peau, elle la colore temporairement. Dans le cas où la solution contient de la Dihydroxyacétone, la peau prend une coloration brune rappelant distinctivement un hâle comparable à un bronzage.

La solution antiseptique selon l'invention fait apparaître rapidement et/ou de façon retardée une coloration de la peau.

Pour les solutions capables de colorer rapidement la peau, comme par exemple les solutions contenant un sel d'argent, de la Ninhydrine ou une encre incolore fluorescente, le temps nécessaire pour que la peau se colore après application de la solution selon l'invention est généralement compris entre 1 et 10 minutes.

Ce délai correspond à la durée requise pour obtenir l'action complète des agents antiseptiques. De ce fait, la visualisation de la couleur sur la peau permet donc à la fois de délimiter la zone aseptisée et de témoigner que les antiseptiques ont bien développé leurs activités antimicrobiennes recherchées.

Pour les solutions capables de colorer la peau de façon retardée comme les solutions contenant de la Dihydroxyacétone, le temps nécessaire pour que la peau se colore après application de la solution selon l'invention est généralement compris entre 1 et 5 heure(s). Le fait que la coloration de la peau ne soit pas instantanée n'est pas un problème pour les asepsies réalisées en blocs opératoires, ces procédures étant effectuées par plusieurs personnes, elles sont de fait autocontrôlées.

La coloration peut subsister plusieurs heures, voire quelques jours ce qui permet notamment de montrer aux patients qui s'appliquent eux-mêmes le produit ou après une opération, que l'asepsie a bien été réalisée afin de prévenir toute maladie nosocomiale. Cette coloration retardée ou prolongée permet donc d'apporter au chirurgien une garantie juridique que le travail a bien été réalisé.

La solution selon l'invention est donc utile pour le marquage de la peau justifiant de la réalisation d'une désinfection. Elle peut être utilisée comme marqueur visuel immédiat et/ou retardé de la réalisation d'une désinfection sur la peau.

Selon un autre avantage, la peau colorée par la solution antiseptique retrouve progressivement sa teinte naturelle, après quelques jours, ce sans aucune intervention ou phase de nettoyage.

De même, la solution antiseptique selon l'invention est incolore et ne peut donc pas tacher le matériel et le mobilier opératoire contrairement aux solutions existantes, lesquelles exigent des produits spécifiques pour éliminer les tâches colorées.

### EXEMPLES

La présente invention peut être illustrée par des exemples non limitatifs de solutions antiseptiques et de procédés de préparation.

### Exemple 1 : Chlorhexidine gluconate / Dihydroxyacétone

La solution selon l'invention peut se présenter sous forme d'une solution comprenant :
- 0,5% de Chlorhexidine digluconate,
- 70% d'alcool éthylique,
- 3% de Dihydroxyacétone, et
- 26,5% d'eau.

Le procédé de préparation pour un lot de 10 litres de solution est réalisé comme suit.

Dans une cuve on introduit 2L d'eau purifiée et 7kg d'alcool éthylique, puis on mélange la préparation pendant 10 minutes environ à l'aide d'un agitateur.

On introduit ensuite sous agitation la Dihydroxyacétone et on agite la préparation durant 5 à 10 minutes environ jusqu'à dissolution complète.

On introduit la Chlorhexidine digluconate sous agitation et on agite 5 à 10 minutes environ jusqu'à dissolution complète.

Enfin, lorsque la solution est limpide, on filtre la préparation sur un filtre membrane de 0,22 microns.

### Exemple 2 : Chlorhexidine digluconate / Dihydroxyacétone

La solution selon l'invention peut se présenter sous forme d'une solution comprenant :
- 0,5% de Chlorhexidine digluconate,
- 70% d'alcool isopropylique,
- 3% de Dihydroxyacétone, et
- 26,5% d'eau.

Le procédé de préparation pour un lot de 10 litres de solution est réalisé comme suit.

Dans une cuve on introduit 2L d'eau purifiée et 7kg d'alcool isopropylique, puis on mélange la préparation pendant 10 minutes environ à l'aide d'un agitateur.

On introduit ensuite sous agitation la Dihydroxyacétone et on agite la préparation durant 5 à 10 minutes environ jusqu'à dissolution complète.

On introduit la Chlorhexidine digluconate sous agitation et on agite 5 à 10 minutes environ jusqu'à dissolution complète.

Enfin, lorsque la solution est limpide, on filtre la préparation sur un filtre membrane de 0,22 microns.

### Exemple 3 : Hexamidine / Dihydroxyacétone

La solution selon l'invention peut se présenter sous forme d'une solution comprenant :
- 0,5% d'Hexamidine,
- 70% d'alcool éthylique,
- 5% de Dihydroxyacétone, et
- 24,5% d'eau.

Le procédé de préparation pour un lot de 10 litres de solution est réalisé comme suit.

Dans une cuve on introduit 2L d'eau purifiée et 7kg d'alcool éthylique, puis on mélange la préparation pendant 10 minutes environ à l'aide d'un agitateur.

On introduit ensuite sous agitation la Dihydroxyacétone et on agite la préparation durant 5 à 10 minutes environ jusqu'à dissolution complète.

On introduit l'Hexamidine sous agitation et on agite 5 à 10 minutes environ jusqu'à dissolution complète.

Enfin, lorsque la solution est limpide, on filtre la préparation sur un filtre membrane de 0,22 microns.

### Exemple 4: Chlorhexidine digluconate/ Chlorure de Benzalkonium/ Dihydroxyacétone

La solution selon l'invention peut se présenter sous forme d'une solution comprenant :
- 0,5% de Chlorhexidine,
- 0,05% de chlorure de Benzalkonium,
- 70% d'alcool éthylique,
- 5% de Dihydroxyacétone, et
- 24,45% d'eau.

Le procédé de préparation pour un lot de 10 litres de solution est réalisé comme suit.

Dans une cuve on introduit 2L d'eau purifiée et 7kg d'alcool éthylique, puis on mélange la préparation pendant 10 minutes environ à l'aide d'un agitateur.

On introduit ensuite sous agitation la Dihydroxyacétone et on agite la préparation durant 5 à 10 minutes environ jusqu'à dissolution complète.

On introduit la Chlorhexidine digluconate sous agitation et on agite 5 à 10 minutes environ jusqu'à dissolution complète.

On introduit le chlorure de Benzalkonium sous agitation et on agite 5 à 10 minutes environ jusqu'à dissolution complète.

Enfin, lorsque la solution est limpide, on filtre la préparation sur un filtre membrane de 0,22 microns.

### Exemple 5: Chlorhexidine gluconate/Ninhydrine

La solution selon l'invention peut se présenter sous forme d'une solution comprenant :
- 0,5% de Chlorhexidine digluconate,
- 70% d'alcool éthylique,
- 0,2% de Ninhydrine, et
- 29,3% d'eau

### Exemple 6: Chlorhexidine gluconate/nitrate d'argent

La solution selon l'invention peut se présenter sous forme d'une solution comprenant :
- 0,5% de Chlorhexidine digluconate,
- 70% d'alcool éthylique,
- 0,5% de nitrate d'argent, et
- 29% d'eau.

Bien entendu, l'invention n'est évidemment pas limitée aux exemples représentés et décrits ci-dessus.

## Revendications

1. Solution antiseptique incolore à usage cutané comprenant au moins un principe actif antiseptique, de l'alcool choisi parmi l'alcool éthylique et l'alcool isopropylique, et au moins une substance incolore capable d'induire une coloration de la peau choisie parmi la Dihydroxyacétone, la Ninhydrine ou un de ses sels, les sels d'argent, la D.F.O (1,8-diaza-9-fluorénone) et les encres fluorescentes.

2. Solution antiseptique incolore à usage cutané selon la revendication 1, **caractérisée en ce que** la concentration en alcool dans la solution est comprise entre 30 et 85%.

3. Solution antiseptique incolore à usage cutané selon l'une des précédentes revendications, **caractérisée en ce que** la substance capable d'induire une coloration de la peau est la Dihydroxyacétone présente à une concentration comprise entre 0,1 et 10%.

4. Solution antiseptique incolore à usage cutané selon l'une des précédentes revendications, **caractérisée en ce que** la substance capable d'induire une coloration de la peau est la Ninhydrine présente à une concentration comprise entre 0,05 et 2%.

5. Solution antiseptique incolore à usage cutané selon l'une des précédentes revendications, **caractérisée en ce que** la substance capable d'induire une coloration de la peau est un sel d'argent présent à une concentration comprise entre 0,1 et 2%.

6. Solution antiseptique incolore à usage cutané selon l'une des précédentes revendications, **caractérisée en ce que** la substance capable d'induire une coloration de la peau est la D.F.O (1,8-diaza-9-fluorénone) présente entre 0,1 et 5%.

7. Solution antiseptique incolore à usage cutané selon l'une quelconque des précédentes revendications, **caractérisée en ce que** le principe actif antiseptique est présent à une concentration comprise entre 0,05 et 5%.

8. Solution antiseptique incolore à usage cutané selon l'une des précédentes revendications, **caractérisée en ce que** le principe actif antiseptique est choisi parmi la Chlorhexidine base, l'Hexamidine, le Benzalkonium ou un de leurs sels.

9. Procédé de préparation d'une solution antiseptique incolore à usage cutané selon l'une des précédentes revendications, comprenant les étapes suivantes :
- mélanger de l'eau purifiée et de l'alcool éthylique ou de l'alcool isopropylique,
- ajouter une substance incolore capable d'induire une coloration de la peau choisie parmi la Dihydroxyacétone, la Ninhydrine ou un de ses sels, les sels d'argent, la D.F.O (1,8-diaza-9-fluorénone) et les encres fluorescentes, et agiter jusqu'à dissolution complète, et
- ajouter au moins un principe actif antiseptique et agiter jusqu'à dissolution complète.

10. Utilisation d'une solution selon l'une des revendications 1 à 8, comme marqueur visuel immédiat et/ou retardé de la réalisation d'une désinfection sur la peau.

## Patentansprüche

1. Farblose antiseptische Lösung zur Verwendung auf der Haut mit wenigstens einem antiseptischen Wirkstoff, mit einem Alkohol, der unter Ethylalkohol und Isopropylalkohol ausgewählt ist, und mit wenigstens einer farblosen Substanz, die dazu geeignet ist, eine Einfärbung der Haut hervorzurufen, und die aus Dihydroxyaceton, Ninhydrin oder eines ihrer Salze, DFO (1,8-Diaza-9-fluorenon) und fluoreszierenden Tinten ausgewählt ist.

2. Farblose antiseptische Lösung zur Verwendung auf der Haut nach Anspruch 1, **dadurch gekennzeichnet, dass** die Konzentration an Alkohol in der Lösung zwischen 30 und 85% liegt.

3. Farblose antiseptische Lösung zur Verwendung auf der Haut nach einem der vorhergehenden Ansprüche, **dadurch gekennzeichnet, dass** die Substanz, die dazu geeignet ist, eine Einfärbung der Haut hervorzurufen, Dihydroxyaceton mit einer Konzentration zwischen 0,1 und 10% ist.

4. Farblose antiseptische Lösung zur Verwendung auf der Haut nach einem der vorhergehenden Ansprüche, **dadurch gekennzeichnet, dass** die Substanz, die in der Lage ist, eine Einfärbung der Haut hervorzurufen, Ninhydrin mit einer Konzentration zwischen 0,05 und 2% ist.

5. Farblose antiseptische Lösung zur Verwendung auf der Haut nach einem der vorhergehenden Ansprüche, **dadurch gekennzeichnet, dass** die Substanz, die in der Lage ist, eine Einfärbung der Haut hervorzurufen, ein Silbersalz mit einer Konzentration zwischen 0,1 und 2% ist.

6. Farblose antiseptische Lösung zur Verwendung auf der Haut nach einem der vorhergehenden Ansprüche, **dadurch gekennzeichnet, dass** die Substanz, die dazu in der Lage ist, eine Einfärbung der Haut hervorzurufen, DFO (1,8-Diaza-9-fluorenon) mit 0,1 bis 5% ist.

7. Farblose antiseptische Lösung zur Verwendung auf der Haut nach einem der vorhergehenden Ansprüche, **dadurch gekennzeichnet, dass** der antiseptische Wirkstoff mit einer Konzentration zwischen 0,05 und 5% enthalten ist.

8. Farblose antiseptische Lösung zur Verwendung auf der Haut nach einem der vorhergehenden Ansprüche, **dadurch gekennzeichnet, dass** der antiseptische Wirkstoff unter Chlorhexidinbase, Hexamidin, Benzalkonium oder einer ihrer Salze ausgewählt ist.

9. Verfahren zur Herstellung einer farblosen antiseptischen Lösung zur Verwendung auf der Haut nach einem der vorhergehenden Ansprüche mit den folgenden Verfahrensschritten:
- Mischen von gereinigtem Wasser und Ethylalkohol oder Isopropylalkohol,
- Hinzufügen einer farblosen Substanz, die in der Lage ist, eine Einfärbung der Haut hervorzurufen, ausgewählt aus Dihydroxyaceton, Ninhydrin oder eine ihrer Salze, den Silbersalzen, DFO (1,8-Diaza-9-Fluorenon) und den fluoreszierenden Tinten, und Bewegen bis zur vollständigen Auflösung, und
- Hinzufügen wenigstens eines antiseptischen Wirkstoffs und Bewegen bis zur vollständigen Auflösung.

10. Verwendung einer Lösung nach einem der Ansprüche 1 bis 8 als sofortige und/oder verzögerte visuelle Markierung der Bewerkstelligung einer Desinfektion auf der Haut.

## Claims

1. A colourless antiseptic solution for skin use comprising at least one antiseptic active ingredient, alcohol chosen from ethyl alcohol and isopropyl alcohol, and at least one colourless substance capable of causing colouring of the skin chosen from dihydroxyacetone, ninhydrin or one of the salts thereof, silver salts, DFO (1,8-diaza-9-fluorenone) and fluorescent inks.

2. A colourless antiseptic solution for skin use according to claim 1, **characterised in that** the alcohol concentration in the solution is between 30% and 85%.

3. A colourless antiseptic solution for skin use according to one of the preceding claims, **characterised in that** the substance capable of causing colouring of the skin is dihydroxyacetone present at a concentration of between 0.1% and 10%.

4. A colourless antiseptic solution for skin use according to one of the preceding claims, **characterised in that** the substance capable of causing colouring of the skin is ninhydrin present at a concentration of between 0.05% and 2%.

5. A colourless antiseptic solution for skin use according to one of the preceding claims, **characterised in that** the substance capable of causing colouring of the skin is a silver salt present at a concentration of between 0.1% and 2%.

6. A colourless antiseptic solution for skin use according to one of the preceding claims, **characterised in that** the substance capable of causing colouring of the skin is DFO (1,8-diaza-9-fluorenone) present at a concentration of between 0.1% and 5%.

7. A colourless antiseptic solution for skin use according to any one of the preceding claims, **characterised in that** the antiseptic active ingredient is present at a concentration of between 0.05% and 5%.

8. A colourless antiseptic solution for skin use according to one of the preceding claims, **characterised in that** the antiseptic active ingredient is chosen from chlorhexidine base, hexamidine, benzalkonium or one of the salts thereof.

9. A method for preparing a colourless antiseptic solution for skin use according to one of the preceding claims, comprising the following steps:
- mixing purified water and ethyl alcohol or isopropyl alcohol,
- adding a colourless substance capable of causing colouring of the skin chosen from dihydroxyacetone, ninhydrin or one of the salts thereof, silver salts, DFO (1,8-diaza-9-fluorenone) and fluorescent inks, and stirring to complete dissolution, and
- adding at least one antiseptic active principle and stirring to complete dissolution.

10. Use of a solution according to one of claims 1 to 8, as an immediate and/or delayed visual marker of the carrying out of disinfection on the skin.
